# EUROPÄISCHE PATENTANMELDUNG

(11) **EP 1 480 150 A2**
(43) Veröffentlichungstag der Anmeldung: **24.11.2004**
(21) Anmeldenummer: 04010584.3
(22) Anmeldetag: 04.05.2004
(51) Int. Cl.: G06F 19/00

(54) **Verfahren zur Verarbeitung von Datensätzen, die Therapiehinweise umfassen**

(30) Priorität: 20.05.2003 DE 10322687
(71) Anmelder: SIEMENS AKTIENGESELLSCHAFT, 80333 München (DE)
(72) Erfinder: Abraham-Fuchs, Klaus, 91058 Erlangen (DE); Rumpel, Eva Dr., 91052 Erlangen (DE)

(57) **Zusammenfassung**

Es wird ein Verfahren zur Referenzierung von Therapiehinweisen (3) umfassenden Datensätzen (D) angegeben, bei dem jeder Datensatz (D) eines Therapiehinweises (3) ein eindeutiges Ordnungsmerkmal (4) umfasst, das ebenfalls zusammen mit einer Therapieinformation (5) die auf dem Therapiehinweis (3) basiert gespeichert wird und wobei im Falle einer Aktualisierung eines Therapiehinweises (3) die Gesamtheit der Therapieinformationen (5) im Hinblick auf das Ordnungsmerkmal (4) des aktualisierten Therapiehinweises (3) durchsucht wird und zu jeder dabei gefundenen Therapieinformation (5) eine die Änderung des zugrundeliegenden Therapiehinweises (3) anzeigende Meldung ausgegeben wird.

## Beschreibung

Die Erfindung betrifft ein Verfahren zur Verarbeitung von Therapiehinweisen, insbesondere von so genannten medizinische Leitlinien umfassenden Datensätzen. Solche Datensätze werden im Folgenden kurz selbst als Therapiehinweis bezeichnet. Die Datensätze können sowohl Daten, also Therapiehinweise, als auch ausführbaren Code, also Softwarefragmente z. B. in Form von Expertenregeln, umfassen.

In den letzten Jahren gewinnen zunehmend so genannte medizinische Leitlinien (im englischen Sprachraum als Medical Practise Guidelines" oder "Clinical Practise Guidelines"), kurz Leitlinie oder Guideline genannt, an Bedeutung. Leitlinien sind diagnostische und/oder therapeutische Handlungs- und Entscheidungsempfehlungen (im weiteren allgemein und zusammenfassend als Therapiehinweise bezeichnet) an den Arzt, die von übergeordneten und allgemein anerkannten Gremien der Ärzteschaft in einem breiten Konsens erarbeitet wurden. Naturgemäß müssen solche Therapiehinweise ständig dem neuesten Stand des Wissens angepasst werden, und unterliegen somit einem ständigen, wenn auch meist lang- oder mittelfristigen Wandel. Typischerweise werden Therapiehinweise in Intervallen von wenigen Jahren neu überarbeitet und angepasst. Ein Arzt, der Patienten bei der Diagnose- und Therapieentscheidung gemäß solcher etablierter Therapiehinweise behandelt, steht nun vor der Herausforderung, sich zeitnah über eine Änderung der Therapiehinweise zu informieren und jeweils den neuesten Stand zu berücksichtigen.

Da sich insbesondere Therapieprozesse über Monate und Jahre erstrecken können, ist es eine noch größere Herausforderung an den Arzt, zum Zeitpunkt der Änderung eines Therapiehinweises den Überblick zu haben, welche seiner Patienten sich derzeit in einer laufenden Behandlung gemäß der veralteten Version des Therapiehinweises befinden, und bei welchen dieser Patienten die neue Version des Therapiehinweises eine Änderung der Therapie erforderlich macht.

Zur Lösung dieser Probleme sind nach bestem Wissen der Anmelderin im Stand der Technik bisher keine automatisch ablauffähigen Verfahren bekannt geworden. Zudem ist der breite Einsatz von Therapiehinweisen insbesondere in Form von Leitlinien in der Medizin erst im Entstehen begriffen.

Der Erfindung liegt daher die Aufgabe zugrunde, ein Verfahren zur Verarbeitung von Daten eines Therapiehinweise umfassenden Datensatzes anzugeben, welches eine möglichst schnelle und einfache Suche und Analyse eines einzelnen Datensatzes ermöglicht. Dabei ist eine möglichst einfache und sichere Verwendung zentral erstellter und bereit gestellter Therapiehinweise, wie z. B. der oben genannten Leitlinien, anzugeben. Diese Aufgabe umfasst zwei Aspekte, nämlich zum einen die Notwendigkeit einer eindeutigen Referenzierbarkeit von Datensätzen mehrerer Therapiehinweise zum sicheren Auffinden eines Datensatzes eines einzelnen Therapiehinweises entsprechend vorgegebener Kriterien. Der weitere Aspekt dieser Aufgabe bezieht sich auf eine möglichst einfache und sichere Verwendung der Therapiehinweise anhand der Datensätze in medizinischen Institutionen und dabei insbesondere auf die Integration sich ändernder oder aktualisierter Therapiehinweise in laufende Therapien.

Der erste Aspekt dieser Aufgabe wird erfindungsgemäß mit den Merkmalen des Anspruchs 1 gelöst. Dazu ist ein Verfahren zur Verarbeitung von Daten eines Therapiehinweise umfassenden Datensatzes, insbesondere zur Referenzierung eines Therapiehinweise umfassenden Datensatzes vorgesehen, wobei eine erste Datenbank eine Anzahl von Datensätzen mehrerer Therapiehinweise umfasst, wobei jedem Datensatz eines Therapiehinweises in Art eines Steuerzeichens ein eindeutiges Ordnungsmerkmal zugeordnet wird und wiederum dem Ordnungsmerkmal in Art eines Steuerzeichens eine Versionskennung und ggf. ein Verwendungshinweis zugeordnet wird, wobei anhand mindestens eines Steuerzeichens, insbesondere anhand des Ordnungsmerkmals und/oder der Versionskennung, derjenige Therapiehinweis automatisch ausgewählt und referenziert sowie ausgegeben wird, dessen Versionskennung je nach Vorgabe die aktuellste oder eine vergangene oder eine vorherige Version darstellt.

Das jedem Therapiehinweis zugeordnete Ordnungsmerkmal in dem betreffenden Datensatz ermöglicht eine eindeutige Referenzierung des jeweiligen Therapiehinweises. Dabei wird dem Ordnungsmerkmal vorteilhafterweise neben dem Referenzierungscode auch eine Information über eine Versionskennung zugeordnet. Anhand der Versionskennung wird derjenige Therapiehinweis identifizierbar, der je nach Vorgabe die aktuellste oder eine vergangene oder eine vorherige Version darstellt. Das Ordnungsmerkmal kann zusätzlich oder alternativ so aufgebaut sein, dass es möglich ist, alle Therapiehinweise die sich auf gleiche medizinische Fragestellungen beziehen, zu identifizieren. Dazu umfasst das Ordnungsmerkmal, das z. B. als Zeichenkette, also in Form eines Textstrings mit vorgegebenem Format realisiert ist, ab einer bestimmten Position des Textstrings als Verwendungshinweis einen Code, insbesondere einen alphanumerischen Code, der die medizinische Fragestellung eindeutig kennzeichnet. Durch Abfrage des Verwendungshinweises können sämtliche Therapiehinweise zu einer gleichen medizinischen Fragestellung ermittelt werden. Wenn auch der Verwendungshinweis selbst nach einem vorgegebenem Format gebildet ist, ist es vorteilhaft möglich, medizinische Fragestellungen in Fallgruppen und Unterfallgruppen usw. zu unterteilen. Damit ist eine noch spezifischer ausgerichtete Identifizierung von Therapiehinweisen für bestimmte medizinische Fragestellungen möglich. Andererseits ist es z. B. auch möglich, wenn nur nach bestimmten Fallgruppen gesucht wird, also unter Außer-Acht-Lassen des restlichen Verwendungshinweises, Parallelitäten in Therapiehinweisen für grundsätzlich unterschiedliche medizinische Fragestellungen zu erkennen.

Des Weiteren ist bei einem Verfahren zur Referenzierung eines Therapiehinweise umfassenden Datensatzes eine erste und eine zweite Datenbank vorgesehen. Die erste Datenbank umfasst eine Anzahl von Therapiehinweisen, wobei jedem Therapiehinweis ein eindeutiges Ordnungsmerkmal zugeordnet wird. Die zweite Datenbank umfasst eine Anzahl von Datensätzen, die Therapieinformationen zu einer individuellen Therapie umfassen. Die Datensätze von Therapieinformationen umfassen dabei Daten, also Therapieinformationen, beispielsweise über Patienten und/oder diagnostische und/oder therapeutische Mittel und/oder andere für eine Therapie erforderliche Ressourcen. Die Therapieinformationen umfassenden Datensätze selbst werden im weiteren kurz als Therapieinformation bezeichnet. Für das Verfahren ist zweckmäßigerweise vorgesehen, dass zu einzelnen oder mehreren Therapieinformationen eines Datensatzes in Art eines Steuerzeichens das Ordnungsmerkmal desjenigen Therapiehinweises abgelegt wird, auf dem die Therapie basiert.

Zusätzlich wird vorzugsweise bei einer Änderung oder einer Aktualisierung eines Therapiehinweises zunächst der geänderte oder aktualisierte Therapiehinweis als neuer Therapiehinweis in die erste Datenbank zusammen mit einem eindeutigen Ordnungsmerkmal hinterlegt, wobei daraufhin die zweite Datenbank im Hinblick auf Therapieinformationen mit dem Ordnungsmerkmal des korrespondierenden alten Therapiehinweises, auf dem der neue Therapiehinweis basiert, durchsucht wird und dass schließlich zu jedem in der zweiten Datenbank gefundenen veralteten Ordnungsmerkmal und diesem zugrunde liegenden veralteten Therapiehinweis eine Meldung generiert wird. Optional kann für jeden neuen Therapiehinweis unabhängig von der bisherigen Verwendung des veralteten, ersetzten oder ergänzten Therapiehinweises eine Meldung generiert werden, so dass die Benutzer von erster und/oder zweiter Datenbank jederzeit über medizinische Fortschritte informiert sind. Diese Information kann anhand eines entsprechend strukturierten Ordnungsmerkmals auf Benutzer bestimmter Fachrichtungen ausgerichtet werden.

Die Erfindung geht von der Erkenntnis aus, dass parallel und unabhängig von der Einführung von Leitlinien in der Medizin derzeit eine starke Ausweitung des Einsatzes moderner Informations- und Kommunikationstechnologie im Gesundheitswesen stattfindet. Der Einsatz elektronischer Datenverarbeitung im Krankenhaus (z. B. HIS = Hospital Information System, RIS = Radiology Information System, PACS = Picture Archive Communication System, LIS = Laboratory Information System) und in der Arztpraxis (Praxisverwaltungssoftware, elektronische Patientenakte) wird mehr und mehr üblich. Von einem folgenden Entwicklungsschritt wird allgemein erwartet, dass eine Vernetzung dieser Software und Datenbanken über die Institutionen des Gesundheitswesens (Kliniken, Arztpraxen, Therapeutenpraxen etc.) hinweg stattfinden wird. Damit wird die Möglichkeit für ein "Vernetztes Gesundheitswesen", zuerst auf nationaler oder regionaler Ebene und später global geschaffen. Diese Entwicklung bietet die Basis zum Einsatz der beiden Aspekte der Erfindung.

Hinsichtlich des ersten Aspektes der oben genannten Aufgabe besteht eine vorteilhafte Ausgestaltung der erfinderischen Lösung darin, dass dem Ordnungsmerkmal ein die Freigabe des jeweiligen Therapiehinweises anzeigendes Freigabeattribut zugeordnet wird. Hierdurch können in die erste Datenbank bereits Therapiehinweise eingestellt werden, die sich z. B. noch in der Erprobung befinden. Interessierte Ärzte oder Therapeuten können sich jederzeit über Fortschritte auf bestimmten Behandlungsgebieten informieren. Das heißt, Freigabe eines Therapiehinweises erfolgt durch übergeordnete Gremien, wie z. B. durch einen ärztlichen Fachverband, und ist unabhängig von den anwendenden Institutionen wie Kliniken.

Wenn zusätzlich oder alternativ zu dem Freigabeattribut ein Aktivierungsattribut erzeugt und mit dem Therapiehinweis verknüpft wird, ist es möglich, einzelne Therapiehinweise, also z. B. veraltete oder überholte Therapiehinweise, gegen eine weitere Verwendung zu sichern. Hierzu wird für den betreffenden Therapiehinweis das zugehörige Aktivierungsattribut aktiviert oder deaktiviert. Das Aktivierungsattribut wird somit für aktuelle und freigegebene Therapiehinweise aktiviert. Bei veralteten oder überholten Therapiehinweisen wird das betreffende Aktivierungsattribut deaktiviert. Das heißt, ist für einen veralteten oder überholten Therapiehinweis das Aktivierungsattribut rückgesetzt, so kann mittels des deaktivierten Aktivierungsattributs eine Neuanwendung des veralteten Therapiehinweises für eine aktuelle Behandlung oder Therapie unterdrückt werden. Somit bleibt, wenn auch solche Therapiehinweise mit deaktiviertem Aktivierungsattribut in der ersten Datenbank verbleiben, die Historie der Entwicklung des einzelnen Therapiehinweises jederzeit erhalten. Das Aktivierungsattribut erfüllt die Funktion, anzuzeigen, dass Patienten mit einem bestimmten Krankheitsbild bevorzugt gemäß dem Therapiehinweis behandelt werden, sofern nicht fallspezifische medizinische Belange entgegenstehen.

Wenn dem Ordnungsmerkmal zusätzlich oder alternativ ein die aktuelle Benutzung des Therapiehinweises anzeigendes Benutzungsattribut zugeordnet wird, ist jederzeit erkennbar, welche Therapiehinweise aktuell bei einer laufenden Behandlung eines Patienten verwendet werden. Dies ist z. B. für lokale Kopien der ersten Datenbank sinnvoll. Die lokale Kopie, die z. B. auf einem Server einer Klinik vorgehalten wird, braucht nur diejenigen Therapiehinweise zu enthalten, die auch tatsächlich in der Praxis verwendet werden. Des weiteren ist das Benutzungsattribut wichtig, um hinsichtlich benutzter Therapiehinweise eine gezielte Möglichkeit der Information über Therapiehinweise bieten zu können, die den benutzten Therapiehinweis ergänzen, berichtigen oder aktualisieren.

Bevorzugt wird das Benutzungsattribut und/oder das Aktivierungsattribut um ein Datum ergänzt oder mit diesem verknüpft, dadurch wird die Historie der Entwicklung des Therapiehinweises (Aktivierungsattribut) und die Historie der Verwendung des Therapiehinweises (Benutzungsattribut) sinnvoll ergänzt.

Hinsichtlich des ersten Aspektes der oben genannten Aufgabe besteht eine vorteilhafte Ausgestaltung der erfinderischen Lösung darin, dass in der ersten Datenbank oder in einer weiteren dritten Datenbank jedem Therapiehinweis ein die Benutzung dieses Therapiehinweises anzeigendes Benutzungsattribut zugeordnet wird. Die Funktion des Benutzungsattributes ist bereits erläutert worden.

Das Benutzungsattribut wird zweckmäßigerweise der ersten Datenbank zugeordnet. Damit ist für den Betreiber der ersten Datenbank erkennbar, welche Therapiehinweise verwendet werden. Das Benutzungsattribut kann jedoch auch Informationen darüber umfassen, wer (d. h. welcher Arzt oder Therapeut, in welcher Institution, z. B. Klinik) den jeweiligen Therapiehinweis verwendet und wo die Verwendung (Stadt, Bezirk, Region, Land, etc.) stattfindet. Wenn die Handhabung eines damit grundsätzlich beliebig detaillierten Benutzungsattributes für den Betreiber der ersten Datenbank nicht mehr sinnvoll handhabbar ist, kann das Benutzungsattribut auch in eine weitere Datenbank hinterlegt werden. Wenn das Benutzungsattribut in einer dritten Datenbank hinterlegt wird, ist eine sichere und einfache Entkopplung zwischen Therapiehinweisen und Therapieinformationen einerseits und eine bessere Handhabbarkeit des Benutzungsattributes für eine ausgewählte Gruppe von Benutzern, z. B. für bestimmte angeschlossene medizinische Institutionen, bestimmte Ärzte und Therapeuten einer medizinischen Institution, erreicht.

Wenn das Benutzungsattribut alternativ oder zusätzlich in der zweiten Datenbank, also z. B. in einer in einer medizinischen Institution wie einer Klinik vorgehaltenen Datenbank hinterlegt wird, hat diese Institution und jeder Nutzer der zweiten Datenbank jederzeit eine patientenbezogene Übersicht über die benutzten Therapiehinweise.

Das oben Gesagte gilt in analoger Weise auch für das weiter oben bereits erläuterte Freigabeattribut und/oder Aktivierungsattribut, das vorteilhaft in der ersten Datenbank oder in einer weiteren dritten Datenbank jedem Therapiehinweis entweder direkt oder über dessen Ordnungsmerkmal zugeordnet und dort hinterlegt wird.

Vorteilhaft wird das Aktivierungsattribut des neuen Therapiehinweises, das auf dem korrespondierenden alten Therapiehinweis basiert, aktiviert, wobei dabei das Aktivierungsattribut des korrespondierenden veralteten Therapiehinweises gleichzeitig deaktiviert wird. Ein deaktiviertes Aktivierungsattribut zeigt somit an, ob der Therapiehinweis veraltet ist und dass anstelle des veralteten Therapiehinweises ein neuer Therapiehinweis in die erste Datenbank aufgenommen wurde oder der veraltete Therapiehinweis gelöscht bzw. dessen Aktivierungsattribut deaktiviert wurde.

Anhand des gesetzten Benutzungsattributs werden vorteilhafterweise in der zweiten Datenbank noch Therapieinformationen identifiziert, die auf dem veralteten Therapiehinweis basieren. Hierdurch kann der veraltete Therapiehinweis in der ersten Datenbank solange verbleiben, bis Therapien, die auf diesem Therapiehinweis basieren, abgeschlossen sind.

Dem Ordnungsmerkmal ist vorzugsweise eine Bezeichnung des Therapiehinweises und eine Versionsnummer zugeordnet. Dies ermöglicht eine besonders komfortable Identifikation des Therapiehinweises. Die Versionsnummer stellt zudem die Protokollierung der Entwicklung einzelner Therapiehinweise sicher.

Bei dem Therapiehinweis handelt es sich bevorzugt um eine medizinische Leitlinie. Wenn ein Therapiehinweis noch nicht in Form einer Leitlinie formuliert werden kann, handelt es sich bei dem Therapiebinweis um geeignete Vorstufen solcher Leitlinien, also z. B. um Literaturhinweise zum Verweis auf Forschungsberichte und dergleichen. Bei der Therapieinformation handelt es sich bevorzugt um eine (elektronische) Patientenakte ist.

Gemäß einer weiteren vorteilhaften Weiterbildung der Erfindung ist vorgesehen, dass in einer Wissensbasis eine Anzahl von Regeln gespeichert wird, wobei sich jede Regel auf Hinweise zu einem Übergang von einem veralteten Therapiehinweis zu einem neuen Therapiehinweis bezieht und wobei mittels eines Auswahlalgorithmus anhand des veralteten Therapiehinweises und des neuen Therapiehinweises eine der Regeln ausgewählt wird. Diese Wissensbasis ermöglicht es aufgrund der Regeln dem jeweiligen Arzt oder Therapeuten konkrete Vorschläge zu unterbreiten, wie im Falle einer Therapie, die auf einem jetzt veralteten Therapiehinweis basiert, weiter verfahren werden sollte. Die Bandbreite der möglichen Vorschläge reicht von "Therapie auf Basis des veralteten Therapiehinweises fortsetzen" bis zu "Therapie sofort auf neuen Therapiehinweis umstellen".

Dabei wird einerseits die Qualität und der Umfang der Änderungen im Therapiehinweis und andererseits die Dauer der bereits begonnenen Therapie in Betracht gezogen. Daher ist weiter vorteilhaft vorgesehen, dass jeder Therapieinformation zumindest ein die erstmalige Verwendung des zugeordneten Therapiehinweises kennzeichnendes Datum zugeordnet wird und dass die Regel anhand der Dauer der Therapie gemäß dem veralteten Therapiehinweis ausgewählt wird. Bei einer gerade erst begonnenen Behandlung ist eine Umstellung unabhängig von Art und Umfang der Änderungen im Therapiehinweis sinnvoll. Bei einer kurz vor dem Abschluss stehenden Behandlung ist eine Umstellung auf den neuen Therapiehinweis nur in den seltensten Fällen vorgesehen.

Der Vorteil der Erfindung und ihrer Ausgestaltungen besteht insbesondere darin, dass die modernen Kommunikationsmöglichkeiten zur Erschließung und Verfügbarmachung des medizinischen Wissens genutzt werden. Der einzelne Arzt oder Therapeut kann jederzeit auf aktuelles medizinisches Fachwissen zugreifen, das insbesondere bereits in Form von konkreten Therapiehinweisen aufbereitet ist. So können auch Ärzte und Therapeuten mit eher breiter fachlicher Ausrichtung Therapien anwenden, die bisher Spezialisten für bestimmte Fachgebiete vorbehalten waren. Dies verbessert die allgemeine Gesundheitsversorgung. Die Gesundheitsversorgung wird auch dadurch verbessert, dass dem einzelnen Arzt oder Therapeuten bereits bei Behandlungsbeginn erprobte Therapiehinweise zur Verfügung stehen. Daraus und aus der damit einhergehenden geringeren zeitlichen Belastung der einzelnen Ärzte und Therapeuten ergibt sich zudem ein erhebliches Einsparpotential.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen.

Darin zeigen:
- FIG 1: eine erste und eine zweite Datenbank mit Therapiehinweise bzw. Therapieinformationen umfassenden Datensätzen,
- FIG 2: die Aktivierung eines neuen Therapiehinweises, und
- FIG 3: eine Wissensbasis zur automatischen Anpassung von Therapien an geänderte, ergänzte oder aktualisierte Therapiehinweise.

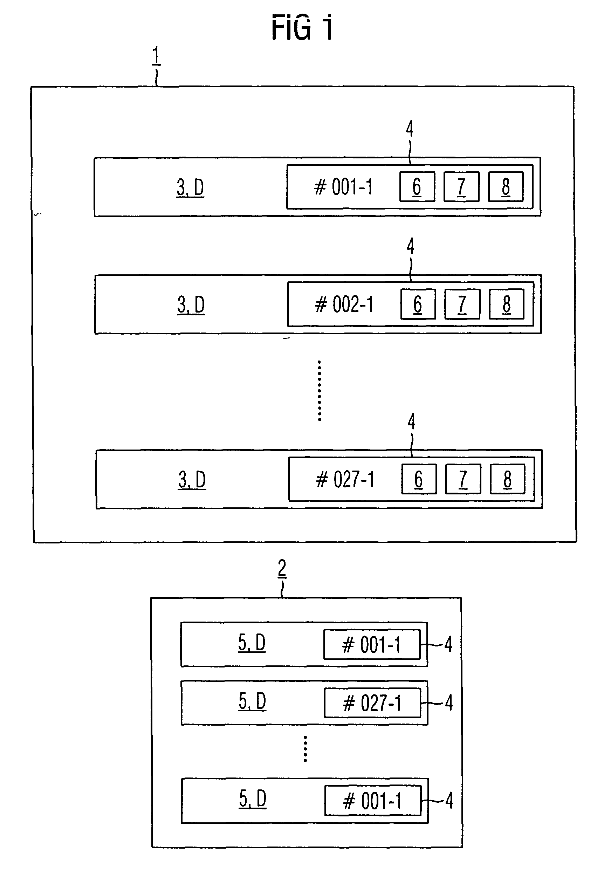

FIG 1 zeigt eine erste Datenbank 1 und eine zweite Datenbank 2. Die erste Datenbank 1 umfasst eine Anzahl von Datensätzen D, die Therapiehinweise 3 umfassen. Diese Datensätze D werden auch als Therapiehinweise 3 umfassende Datensätze D oder kurz als Therapiehinweise 3 bezeichnet. Jeder Therapichinweis 3 weist ein eindeutiges Ordnungsmerkmal 4 auf. Der Inhalt oder der "Wert" des Ordnungsmerkmals 4 ist in der FIG 1 zur besseren Unterscheidbarkeit von Bezugszeichen mit vorangestelltem "#"-Zeichen angegeben. Das Ordnungsmerkmal 4 des ersten, in der ersten Datenbank 1 gespeicherten Therapiehinweises 3 lautet beispielhaft "#001-1", des zweiten "#002-1", des letzten "#027-1".

Die zweite Datenbank 2 umfasst eine Anzahl von Datensätzen D, die Therapieinformationen 5 umfassen. Diese Datensätze D werden auch als Therapieinformationen 5 umfassende Datensätze D oder kurz als Therapieinformationen 5 bezeichnet.

Die Therapieinformationen 5 umfassen als Daten in Form einer elektronischen Akte beispielsweise Patientenakten, aber auch zusätzlich Informationen oder Daten über therapeutische und/oder diagnostische Mittel und Ressourcen. Zu einer Therapieinformation 5 ist das Ordnungsmerkmal 4 desjenigen Therapiehinweises 3 abgelegt, auf dem die dem betreffenden Patienten zugehörige Therapie basiert. Somit beziehen sich die Therapieinformationen 5 unter anderem auch auf eine individuelle, insbesondere personenbezogene Therapie. Entsprechend basiert gemäß den dargestellten Verhältnissen die erste und die letzte Therapieinformation 5, z. B. die erste und die letzte Patientenakte, in der zweiten Datenbank 2 auf dem ersten Therapiehinweis 3 der ersten Datenbank 1 und die zweite Therapieinformation 5 in der zweiten Datenbank 2 auf dem letzten Therapiehinweis 3 der ersten Datenbank 1. Durch die Verknüpfung von Therapieinformation 5 und Therapiehinweis 3 über das Ordnungsmerkmal 4 des Therapiehinweises 3 ist eine jederzeitige Zuordnung von Therapieinformation 5 und Therapiehinweis 3 gewährleistet. Mit anderen Worten: Bei den Therapiehinweisen 3 handelt es sich insbesondere um eine medizinische Leitlinie unter anderem zur Behandlung eines Patienten. Bei den Therapieinformationen 5 handelt es sich insbesondere um eine Patientenakte, insbesondere um eine elektronische Patientenakte.

Zur Identifizierung, ob ein bestimmter Therapiehinweis 3 zur Benutzung freigegeben ist, wird dem Ordnungsmerkmal 4 ein Freigabeattribut 6 zugeordnet. Das Freigabeattribut 6 kann sich auf eine Freigabe des betreffenden Therapiehinweises 3 zur allgemeinen Benutzung oder zur Benutzung in einer bestimmten Institution oder in einer bestimmten Region beziehen. Für derartige Zwecke ist das Freigabeattribut 6 hierarchisch organisiert.

Zur Identifizierung ob ein bestimmter Therapiehinweis 3, insbesondere ein zur Benutzung freigegebener Therapiehinweis 3 in einer medizinischen Institution bei Vorliegen entsprechender Indikationen verwendet werden soll, wird dem Ordnungsmerkmal 4 zusätzlich oder alternativ ein Aktivierungsattribut 7 zugeordnet.

Zur Identifizierung ob ein bestimmter Therapiehinweis 3, insbesondere ein zur Benutzung freigegebener Therapiehinweis 3 tatsächlich benutzt wird, wird dem Ordnungsmerkmal 4 zusätzlich oder alternativ ein Benutzungsattribut 8 zugeordnet.

Nachfolgend wird die Verwendung einzelner Therapiehinweise 3 anhand von Therapieinformationen 5 näher erläutert:

In der ersten Datenbank 1, welche in einer medizinischen Institution oder auf einem zentralen Server gemeinsam für viele Institutionen hinterlegt wird und über an sich bekannte Formen der elektronischen Datenvernetzung, z. B. Internet, (nicht dargestellt) in der Institution oder den Institutionen zur Verfügung steht, ist jedem Therapiehinweis 3 ein eindeutiges Ordnungsmerkmal 4, z. B. eine Nummer, ein Stichwort, ein alphanumerischer Code, zugeordnet. Im Beispiel handelt es sich für die dargestellten Therapiehinweise 3 um den Code "001", "002" und "027". Dieses Ordnungsmerkmal 4 enthält auch eine Information über die Version des Therapiehinweises 3, so dass zwei Therapiehinweise 3, die derselben medizinischen Fragestellung oder Leitlinie zuzuordnen sind, aber historisch in Ihrer Gültigkeit aufeinander folgen, eindeutig als zusammengehörig identifizierbar sind und aus dem Ordnungsmerkmal 4 bzw. aus dessen Versionsinformation auch hervorgeht, in welcher Reihenfolge die unterschiedlichen Therapiehinweis-Versionen in Kraft getreten sind. Die Versionskennung ist im Beispiel mit "-1" (für die erste Version) angegeben.

Besonders günstig ist, wenn das Ordnungsmerkmal 4, insbesondere die Nummer, das Stichwort oder der alphanumerische Code gemäß einem allgemein verbindlichen standardisierten Verfahren gebildet wird. Dies macht jedes Ordnungsmerkmal 4 zu einem eindeutigen Ordnungsmerkmal 4, d. h. jedes Ordnungsmerkmal 4 bezeichnet genau einen Therapiehinweis 3.

In der ersten Datenbank 1 oder ggf. auch in einer weiteren Datenbank wird zu jedem Ordnungsmerkmal 4 eines Therapiehinweises 3 anhand eines zugehörigen Aktivierungsattributs 7 eine Information darüber gespeichert, ob dieser Therapiehinweis 3 veraltet oder aktuell ist und somit in der Institution eingesetzt wird. "In der Institution eingesetzt" heißt hierbei, dass Patienten mit dem entsprechenden Krankheitsbild in der Institution bevorzugt gemäß den Empfehlungen dieses Therapiehinweises 3 behandelt werden, insofern nicht andere fallspezifische medizinische Belange entgegenstehen. Der Eintrag eines Datums, zu dem der Therapiehinweis 3 in Kraft getreten ist, kann die Funktion des Aktivierungsattributes 7 teilweise erfüllen.

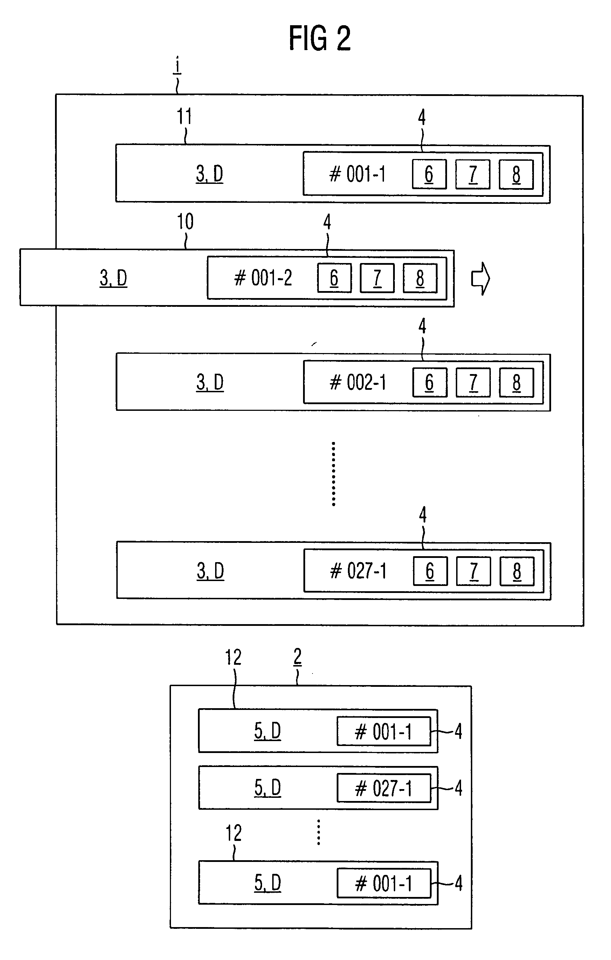

Der Speicherort des Aktivierungsattributes 7 und auch des nachfolgend hinsichtlich seiner Bedeutung noch weiter zu beschreibenden Benutzungsattributes 8 ist grundsätzlich beliebig. Ein Speichern dieser Attribute zusammen mit dem jeweiligen Therapiehinweis 3 (wie in FIG 1 und FIG 2 dargestellt), ist dann vorteilhaft, wenn nur wenige oder einzelne Institutionen auf die erste Datenbank 1 zugreifen oder wenn der Betreiber der ersten Datenbank 1 hierarchisch organisierte Formen dieser Attribute - Aktivierungsattribut 7 und/oder Benutzungsattribut 8 - anbieten kann, so dass auch ein Zugriff einer Vielzahl von Benutzern unterscheidbar möglich ist.

Wenn diese Attribute - Aktivierungsattribut 7 und/oder Benutzungsattribut 8 - nicht in der ersten Datenbank 1 hinterlegt werden, bietet sich eine Hinterlegung in der zweiten Datenbank 2 an. Die zweite Datenbank 2 zerfällt dann quasi in zwei Datenbereiche, nämlich in einen Datenbereich mit Therapieinformationen 5, die mit dem zugrundeliegenden Therapiehinweis 3 über das Ordnungsmerkmal 4 verknüpft sind und in einen weiteren Datenbereich mit Attributen, also dem Freigabe-, Aktivierungs- und/oder Benutzungsattribut 6, 7 bzw. 8, die mit dem zugrundeliegenden Therapiehinweis 3 ebenfalls über das Ordnungsmerkmal 4 verknüpft sind. Nachdem die beiden Datenbereiche der zweiten Datenbank 2 bei der oben skizzierten Ausführungsform unabhängig voneinander sind und eine Zuordnung zum jeweils zugrunde liegenden Therapiehinweis 3 immer über das Ordnungsmerkmal 4 gegeben ist, kann der Datenbereich mit den Attributen - Freigabeattribut 6, Aktivierungsattribut 7 und/oder Benutzungsattribut 8 - auch in einer separaten weiteren Datenbank (nicht dargestellt) realisiert werden. Eine solche separate Datenbank kann für eine Gruppe von Institutionen gemeinsam verwaltet werden. Es entsteht quasi eine "eingeschobene" Hierarchieebene, die dann sinnvoll ist, wenn mehrere Institutionen gemeinsam auf die erste Datenbank 1 zugreifen, eine gemeinsame Hinterlegung und Verwaltung aller Attribute in der ersten Datenbank 1 jedoch nicht oder nur schwer handhabbar ist.

In der zweiten Datenbank 2, z. B. der elektronischen Patientenakte, wird zu Beginn einer medizinischen Therapie (Diagnoseprozess, Therapie, Rehabilitationsmaßnahme, etc.), die gemäß einem bestimmten Therapiehinweis 3 durchgeführt wird, das Ordnungsmerkmal 4 dieses Therapiehinweises 3 und das Datum des Beginns der Therapie gespeichert. Optional oder zusätzlich kann auch die vorhergesehene Dauer oder das vorhergesehen Ende der Therapie gespeichert werden. Alternativ zum Datum des Beginns der Therapie oder auch zusätzlich kann in der ersten Datenbank 1 das Benutzungsattribut 8 beispielsweise auf einen vorgegebenen Wert gesetzt werden, so dass damit anzeigt wird, dass der Therapiehinweis 3 mit diesem Ordnungsmerkmal 4 derzeit in einem aktiven Behandlungsprozess zumindest eines Patienten verwendet wird. Bei Beendigung der Therapie gemäß dem Therapiehinweis 3 wird ein Enddatum eingetragen und/oder das Benutzungsattribut 8 zurückgesetzt.

Nachfolgend wird anhand von FIG 2 die Aktivierung eines neuen Therapiehinweises 10, also eines Therapiehinweises 3, der einen bestehenden Therapiehinweis 3 (= veralteter Therapiehinweis 11) ersetzt, näher erläutert:

Der neue Therapiehinweis 10 wird in die erste Datenbank 1 zusammen mit einem eindeutigen Ordnungsmerkmal 4 - also "#001-2" - aufgenommen. Im dargestellten Beispiel unterscheidet sich das Ordnungsmerkmal 4 des neuen Therapiehinweises 10 vom entsprechenden Ordnungsmerkmal 4 des alten Therapiehinweises 11 nur durch die geänderte Versionskennung ("-2").

Sobald das Aktivierungsattribut 7 des neuen Therapiehinweises 10 auf "aktiviert" gesetzt wird , wird automatisch das Aktivierungsattribut 7 des alten Therapiehinweises 11 auf "deaktiviert" gesetzt.

Die oder jede zweite Datenbank 2 wird im Hinblick auf Therapieinformationen 5 mit dem Ordnungsmerkmal 4 des veralteten Therapiehinweises 11 - also "#001-1" - durchsucht. Wenn in der oder einer zweiten Datenbank 2 eine Therapieinformation 5 mit dem Ordnungsmerkmal 4 des veralteten Therapiehinweises 11 gefunden wird, wird in der ersten Datenbank 1 das Benutzungsattribut 8 des veralteten Therapiehinweises 11 beispielsweise auf einen ersten Wert ("verwendet") zurückgesetzt. Wenn in der oder einer zweiten Datenbank 2 eine Therapieinformation 5 mit dem Ordnungsmerkmal 4 des veralteten Therapiehinweises 11 nicht gefunden wird, wird das Benutzungsattribut 8 des veralteten Therapiehinweises 11 auf einen zweiten Wert ("gestrichen") zurückgesetzt. Der Eintrag eines Datums zu dem die Gültigkeit des Therapiehinweises 3 in der oder für die Institution außer Kraft gesetzt wurde, kann die Funktion des Aktivierungsattributes 7 teilweise ersetzen.

Für sämtliche aufgefundene Therapieinformationen 5 mit dem Ordnungsmerkmal 4 des veralteten Therapiehinweises 11, wird eine Meldung, z. B. eine elektronische Mail an den behandelnden Arzt oder Therapeuten oder dergleichen, erzeugt. Auf diese Weise ist eine zeitnahe und zielgerichtete Information über Änderungen in Therapiehinweisen 3, die in angewandte Therapien einfließen, gewährleistet.

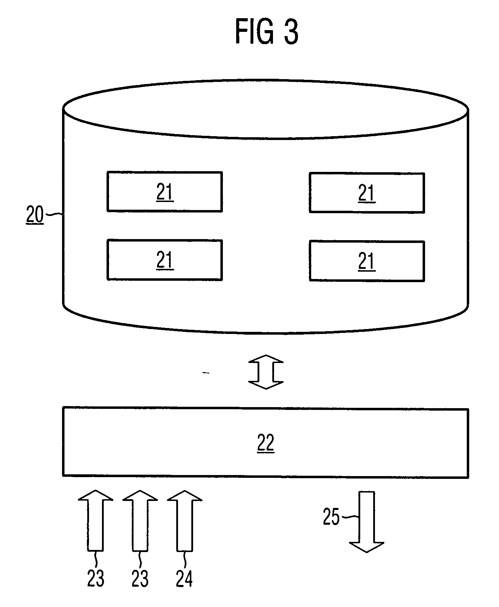

FIG 3 zeigt eine Wissensbasis 20 zur automatischen Anpassung von Therapien an geänderte, ergänzte oder aktualisierte Therapiehinweise 3. In der Wissensbasis 20 sind eine Anzahl von Regeln 21 gespeichert. Jede Regel 21 bezieht sich auf Hinweise zu einem Übergang von einem veralteten Therapiehinweis 11 (FIG 2) zu einem neuen Therapiehinweis 10 (FIG 2). Mittels eines Auswahlalgorithmus 22 wird anhand des veralteten Therapiehinweises 11 und des neuen Therapiehinweises 10 und ggf. anhand von Therapieinformationen 5 eine der Regeln 21 ausgewählt. Der neue und der veraltete Therapiehinweis 10 bzw. 11 und/oder Therapieinformationen 5 wie zugehörige Patientendaten, z. B. das Datum der erstmaligen Verwendung wird dem Auswahlalgorithmus 22 an dafür vorgesehenen Eingängen 23 zugeführt. Vorteilhaft wird in die Auswahl einer Regel 21 auch der Beginn, die Dauer und/oder die Gesamtdauer einer Therapie einbezogen. Diese Daten stehen in der zweiten Datenbank 2 z. B. als Bestandteil der Therapieinformation 5, 12 zur Verfügung. Die Daten werden dem Auswahlalgorithmus 22 an einem dafür vorgesehenen weiteren Eingang 24 zugeführt. Der Auswahlalgorithmus 22 wählt daraufhin aus der Wissensbasis 20 eine Regel 21 zum Übergang vom veralteten Therapiehinweis 11 zum neuen Therapiehinweis 10 für eine Therapieinformation 5, z. B. für eine Patienten aus. An einem Ausgang 25 werden Anweisungen zum Übergang vom veralteten Therapiehinweis 11 zum neuen Therapiehinweis 10 gemäß der ausgewählten Regel 21 ausgegeben.

Damit lässt sich die vorliegende Erfindung zusammenfassend kurz wie folgt darstellen:

Es wird ein Verfahren zur Referenzierung von Therapiehinweise 3 umfassenden Datensätzen D angegeben, bei dem jedem Datensatz D eines Therapiehinweises 3 ein eindeutiges Ordnungsmerkmal 4 zugeordnet wird, das ebenfalls zusammen mit einer Therapieinformation 5, die dem Therapiehinweis 3 zugeordnet wird, gespeichert wird und wobei im Falle einer Aktualisierung eines Therapiehinweises 3 die Gesamtheit der Therapieinformationen 5 im Hinblick auf das Ordnungsmerkmal 4 des aktualisierten Therapiehinweises 3 bzw. 10 durchsucht wird und zu jeder dabei gefundenen Therapieinformation 5 eine die Änderung des zugrundeliegenden Therapiehinweises 3 bzw. 10 anzeigende Meldung ausgegeben wird.

## Patentansprüche

1. Verfahren zur Verarbeitung eines Therapiehinweise (3) umfassenden Datensatzes (D), die in einer ersten Datenbank (1) hinterlegt sind, wobei jedem Datensatz (D) eines Therapiehinweises (3) ein eindeutiges Ordnungsmerkmal (4) und wiederum dem Ordnungsmerkmal (4) eine Versionskennung und ggf. ein Verwendungshinweis zugeordnet werden, wobei anhand des Ordnungsmerkmals (4) und/oder der Versionskennung derjenige Datensatz (D) des betreffenden Therapiehinweises (3) automatisch ausgewählt, referenziert und ausgegeben wird, dessen Versionskennung je nach Vorgabe die aktuellste oder eine vergangene oder eine vorherige Version darstellt.

2. Verfahren nach Anspruch 1, wobei dem Ordnungsmerkmal (4) ein die Freigabe dieses Therapiehinweises (3) anzeigendes Freigabeattribut (6) und/oder ein die Benutzbarkeit dieses Therapiehinweises (3) anzeigendes Aktivierungsattribut (7) zugeordnet wird.

3. Verfahren nach Anspruch 1, wobei dem Ordnungsmerkmal (4) ein die aktuelle Benutzung dieses Therapiehinweises (3) anzeigendes Benutzungsattribut (8) zugeordnet wird.

4. Verfahren nach Anspruch 3, wobei das Benutzungsattribut (8) und/oder das Aktivierungsattribut (7) ein Datum umfasst.

5. Verfahren nach einem der vorhergehenden Ansprüche, bei dem in der zweiten Datenbank (2) zu Beginn einer medizinischen Therapie ein zugrunde liegender Therapiehinweis (3) identifiziert und dessen zugehöriges Ordnungsmerkmal (4) und das Datum des Beginns der Therapie gespeichert werden.

6. Verfahren nach einem der vorhergehenden Ansprüche,
wobei in einer zweiten Datenbank (2), die eine Anzahl von Datensätzen (D) für Therapieinformationen (5) zu einer individuellen Therapie aufweist, jedem Datensatz (D) einer Therapieinformation (5) das Ordnungsmerkmal (4) desjenigen Therapiehinweises (3) zugeordnet wird, auf dem die Therapie basiert.

7. Verfahren nach Anspruch 6, wobei bei einer Änderung oder einer Aktualisierung eines Therapiehinweises (3)
- der geänderte oder aktualisierte Therapiehinweis (3) als neuer Therapiehinweis (10) in Form eines Datensatzes (D) in die erste Datenbank (1) zusammen mit einem eindeutigen Ordnungsmerkmal (4) hinterlegt wird, und
- wobei die zweite Datenbank (2) im Hinblick auf Therapieinformationen (5) mit dem Ordnungsmerkmal (4) des korrespondierenden alten Therapiehinweises (3), auf dem der neue Therapiehinweis (10) basiert, durchsucht wird, und
- zu jedem in der zweiten Datenbank (2) gefundenen veralteten Ordnungsmerkmal (4) eine Meldung generiert wird.

8. Verfahren nach Anspruch 6 oder 7, wobei für jeden neu in die erste Datenbank (1) hinterlegten Therapiehinweis (3) eine entsprechende Meldung generiert wird.

9. Verfahren nach einem der Ansprüche 6 bis 8, wobei in der ersten Datenbank (1) oder in einer weiteren dritten Datenbank jedem Therapiehinweis (3) ein die Benutzbarkeit dieses Therapiehinweises (3) anzeigendes Aktivierungsattribut (7) und/oder ein die Freigabe dieses Therapiehinweises (3) anzeigendes Freigabeattribut (6) zugeordnet und/oder aktiviert wird.

10. Verfahren nach einem der Ansprüche 6 bis 9, wobei in der ersten Datenbank (1) oder in einer weiteren dritten Datenbank jedem Therapiehinweis (3) ein die Benutzung dieses Therapiehinweises (3) anzeigendes Benutzungsattribut (8) zugeordnet und/oder aktiviert wird.

11. Verfahren nach Anspruch 10, wobei das Benutzungsattribut (8) und/oder das Freigabeattribut (6) und/oder das Aktivierungsattribut (7) ein Datum umfasst.

12. Verfahren nach Anspruch 10 oder 11, wobei das Benutzungsattribut (8) gesetzt wird, wenn in der zweiten Datenbank (2) noch Therapieinformationen (5) identifiziert werden, die auf dem veralteten Therapiehinweis (11) basieren.

13. Verfahren nach einem der Ansprüche 6 bis 12, wobei dem Ordnungsmerkmal (4) eine Bezeichnung des Therapiehinweises (3) und eine Versionsnummer zugeordnet wird.

14. Verfahren nach einem der Ansprüche 6 bis 13, wobei in einer Wissensbasis (20) eine Anzahl von Regeln (21) gespeichert ist, wobei sich jede Regel (21) auf Hinweise zu einem Übergang von einem veralteten Therapiehinweis (11) zu einem neuen Therapiehinweis (10) bezieht und wobei mittels eines Auswahlalgorithmus (22) anhand des veralteten Therapiehinweises (11) und des neuen Therapiehinweises (10) sowie anhand von Therapieinformationen (5) eine der Regeln (21) auswählt wird.

15. Verfahren nach Anspruch 14, wobei jeder Therapieinformation (5) zumindest ein die erstmalige Verwendung des zugeordneten Therapiehinweises (3) kennzeichnendes Datum zugeordnet wird und wobei die Regel (21) anhand der Dauer der Therapie gemäß dem veralteten Therapiehinweis (11) ausgewählt wird.

16. Verfahren nach einem der Ansprüche 1 bis 15, wobei der Therapiehinweis (3) eine medizinische Leitlinie und wobei die Therapieinformation (5) eine Patientenakte und/oder Informationen über therapeutische und/oder diagnostische Mittel repräsentiert.

17. Computerprogrammprodukt zur Durchführung eines Verfahrens nach einem der Ansprüche 1 bis 16.
